## Europäisches Patentamt
## European Patent Office
## Office européen des brevets

(19)

(11) Numéro de publication:

**0 187 109**
**A1**

(12)

# DEMANDE DE BREVET EUROPEEN

(21) Numéro de dépôt: 85430043.1

(22) Date de dépôt: 13.12.85

(51) Int. Cl.⁴: **A 61 M 1/16**

(30) Priorité: 14.12.84 FR 8419314

(43) Date de publication de la demande: 09.07.86
Bulletin 86/28

(84) Etats contractants désignés: BE CH DE GB IT LI SE

(71) Demandeur: Issautier, Gérald, Domaine des Alpilles
Immeuble le Saint Rémy 397, Corniche Kennedy,
F-13007 Marseille (FR)

(72) Inventeur: Issautier, Gérald, Domaine des Alpilles
Immeuble le Saint Rémy 397, Corniche Kennedy,
F-13007 Marseille (FR)

(74) Mandataire: Azais, Henri et al, c/o CABINET BEAU DE
LOMENIE 14, rue Raphael, F-13008 Marseille (FR)

(54) Dispositif d'hémodialyse permettant de contrôler automatiquement la perte de poids.

(57) L'invention a pour objet un dispositif d'hémodialyse permettant de contrôler automatiquement la perte de poids.

Un dispositif selon l'invention comporte un dialyseur (1), un réservoir de dialysat (5), une cartouche (7) de régénération du dialysat et une pompe péristaltique (3) à un seul rotor qui fait circuler le sang et le dialysat dans deux tubes séparés. Le conduit de dialysat (10) allant du réservoir (5) au dialyseur (1), comporte un clapet de non-retour (11) et une canalisation (16), branchée en dérivation en aval dudit clapet, passant à travers une pompe péristaltique (17), qui permet de faire varier la pression du dialysat dans le dialyseur. La pompe (17) est commandée automatiquement, soit par une boucle de régulation qui compare la valeur mesurée de la pression transmembranaire à une valeur de consigne, soit par une autre boucle de régulation qui compare la perte de poids du réservoir (5) par unité de temps mesurée par un capteur (21) à une valeur de consigne.

Une application est la construction d'un appareil d'hémodialyse facile à transporter et à utiliser.

Dispositif d'hémodialyse permettant de contrôler automatiquement la perte de poids.

La présente invention a pour objet des dispositifs d'hémodialyse permettant de contrôler automatiquement la perte de poids.

On rappelle brièvement qu'un dispositif d'hémodialyse comporte un dialyseur qui est séparé en deux compartiments par une membrane semi-perméable, de part et d'autre de laquelle on fait circuler le sang d'un patient atteint d'insuffisance rénale et un liquide de dialyse, de sorte que certaines substances toxiques qui se trouvent dans le sang et qui auraient dû être épurées par les reins, passent à travers la membrane et sont évacuées par le liquide de dialyse.

On connaît des appareils de dialyse dans lesquels le liquide de dialyse chargé d'impuretés est rejeté. Ces appareils comportent un générateur de liquide de dialyse qui est très encombrant.

On connaît également des appareils de dialyse comportant une cartouche d'épuration du liquide de dialyse, de sorte que ce liquide peut être recyclé. Les appareils de ce deuxième type sont moins encombrants et peuvent être plus facilement installés au domicile d'un patient.

La présente invention concerne des appareils de ce deuxième type qui sont facilement transportables.

Au cours d'une séance de dialyse, le patient perd un poids égal à la quantité de liquide chargé d'impuretés qui est passée à travers la membrane de dialyse.

Comme la durée d'une dialyse est généralement déterminée, la perte de poids totale dépend de la perte de poids par unité de temps, qui dépend elle-même de la perméabilité de la membrane, qui est une donnée de construction, et de la pression transmembranaire, c'est-à-dire de la différence algébrique entre la pression relative toujours positive du sang et la pression relative négative ou nulle du liquide de dialyse, de part et d'autre de la membrane.

A ce jour, pour régler la pression transmembranaire, on ajuste généralement la pression sanguine au moyen d'une pince qui est située sur le tube de retour vers la veine du patient et que l'on serre plus ou moins. Ce réglage est effectué manuellement. Il expose le circuit sanguin à une surpression dangereuse.

Un objectif de la présente invention est de procurer un dispositif de dialyse qui comporte des moyens permettant d'obtenir automatiquement une perte de poids déterminée par unité de temps et donc une perte de poids totale déterminée au cours d'une séance de dialyse de durée donnée.

Un autre objectif de la présente invention est de procurer un dispositif de dialyse peu encombrant et portatif, qui peut être utilisé à domicile et transporté par un patient dans ses déplacements.

Un dispositif d'hémodialyse selon l'invention est du type comportant un dialyseur équipé d'une membrane de dialyse, un réservoir contenant le liquide de dialyse, une cartouche de régénération du liquide de dialyse et des moyens pour faire circuler le sang d'un patient à travers ledit dialyseur et pour faire circuler le liquide de dialyse en circuit fermé entre ledit réservoir, ledit dialyseur et ladite cartouche de régénération.

Les objectifs de l'invention sont atteints au moyen d'un dispositif dans lequel le conduit de liquide de dialyse allant dudit réservoir audit dialyseur comporte un clapet anti-retour et une dérivation qui est branchée sur ledit conduit en aval dudit clapet et qui comporte une première pompe volumétrique à double sens de marche.

Selon un mode de réalisation préférentiel, les moyens pour faire circuler le sang et le liquide de dialyse sont constitués par une même deuxième pompe volumétrique de type péristaltique, dont le rotor écrase deux tubes souples, qui véhiculent respectivement le sang et le liquide de dialyse avec des débits constamment proportionnels entre eux et le tube amenant le sang à l'entrée du dialyseur est connecté sur le refoulement de ladite pompe tandis que le tube qui reprend le liquide de dialyse à la sortie du dialyseur est connecté sur l'aspiration de ladite pompe, de sorte qu'il existe toujours une différence positive entre la pression du sang et la pression du liquide de dialyse, de part et d'autre de la membrane de dialyse.

Un dispositif selon l'invention comporte, de façon connue, un premier capteur de pression qui mesure la pression du sang dans le dialyseur ou à la sortie de celui-ci et un deuxième capteur de pression qui mesure la pression du liquide de dialyse dans le dialyseur ou à la sortie de celui-ci.

Selon un premier mode de réalisation, un dispositif selon l'invention comporte une boucle de régulation qui comporte des moyens pour faire la différence entre les signaux délivrés par lesdits capteurs et un comparateur qui compare cette différence à une valeur de consigne et qui émet un signal proportionnel à l'écart entre cette différence et la valeur de consigne qui agit automatiquement sur le sens et sur la vitesse de rotation de ladite première pompe volumétrique afin de corriger la pression du liquide de dialyse dans le sens qui maintient ladite différence constamment égale à ladite valeur de consigne.

Selon un deuxième mode de réalisation, la cartouche de régénération du liquide de dialyse est placée à l'intérieur du réservoir qui est connecté au reste de l'appareil par des tubes souples et le dispositif comporte un capteur de poids qui mesure la variation de poids dudit réservoir par unité de temps et une boucle de régulation comportant un comparateur qui compare ladite mesure à une valeur de consigne et qui émet un signal proportionnel à l'écart qui agit automatiquement sur le sens et sur la vitesse de rotation de ladite première pompe afin de corriger la pression du liquide de dialyse dans le sens qui maintient la perte de poids mesurée par unité de temps constamment égale à ladite valeur de consigne.

L'invention a pour résultat de nouveaux appareils d'hémodialyse qui sont des appareils de poids et d'encombrement réduit, pouvant être utilisés à domicile ou au cours de déplacements.

Les appareils d'hémodialyse selon l'invention permettent qu'un patient qui les utilise chez lui ou en déplacement, sans être sous le contrôle d'un personnel qualifié, puisse régler lui-même, de façon très simple, la perte de poids totale par séance de dialyse, qui est un facteur très important, grâce aux dispositifs de contrôle automatique de la différence de pression transmembranaire et/ou de la perte de poids par unité de temps qui équipent un appareil selon l'invention.

La pompe péristaltique unique qui fait circuler à la fois le sang et le liquide de dialyse au moyen d'un même rotor, permet d'obtenir, en toutes circonstances, un rapport constant entre le débit de sang et le débit de liquide de dialyse et une pression transmembranaire positive.

Le dispositif composé d'une pompe péristaltique branchée en dérivation en aval d'un clapet de non retour situé sur le tube qui véhicule le liquide de dialyse entre le réservoir de stockage et le dialyseur, permet de contrôler efficacement et automatiquement la pression du liquide de dialyse dans le dialyseur et donc la pression transmembranaire qui est le facteur essentiel de la perte de poids à travers la membrane.

Un appareil selon l'invention peut être équipé d'un microprocesseur qui calcule la valeur de consigne de la perte de poids par unité de temps à partir de deux données qui sont la perte de poids totale et la durée de la dialyse, qui calcule la perte de poids effective par unité de temps à partir d'une mesure de poids total, qui compare les deux valeurs et qui commande automatiquement le sens de marche et la vitesse de la pompe placée en dérivation pour faire varier la pression du liquide de dialyse dans le sens voulu afin que la perte de poids par unité de temps mesurée soit constamment égale à la perte de poids par unité de temps calculée.

On peut également entrer dans le microprocesseur une valeur de consigne de la pression et le microprocesseur calcule la pression transmembranaire effective à partir des pressions du sang et du liquide de dialyse qui lui sont transmises par deux capteurs de pression, il compare à la valeur de consigne et il commande automatiquement le sens de rotation et la vitesse de la pompe placée en dérivation pour maintenir la pression transmembranaire mesurée constamment égale à la valeur de consigne.

La description suivante se réfère aux dessins annexés qui représentent, sans aucun caractère limitatif, un exemple de réalisation préférentiel d'un dispositif d'hémodialyse selon l'invention.

La figure 1 représente schématiquement les éléments essentiels d'un dispositif d'hémodialyse selon l'invention.

La figure 2 est une vue d'ensemble en perspective d'un appareil de dialyse portatif selon l'invention.

La figure 3 est un schéma des circuits électroniques de régulation de la perte de poids du malade.

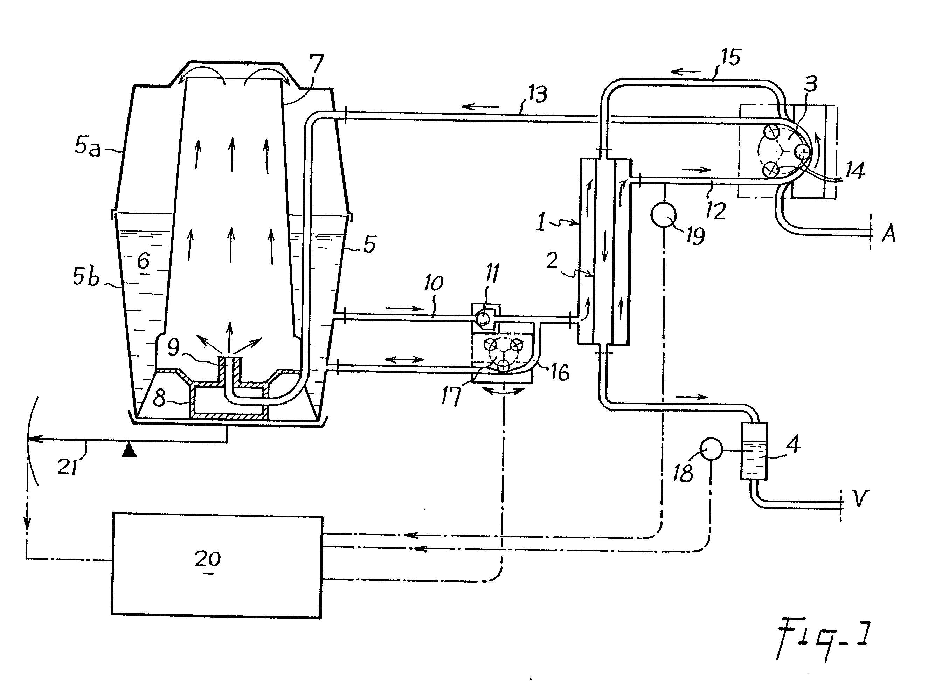

La figure 1 représente un appareil d'hémodialyse, appelé rein artificiel, qui comporte, de façon connue, un dialyseur 1, de tout type connu, qui comporte une membrane de dialyse 2, de tout type connu. Le sang prélevé sur une artère d'un patient A est aspiré par une pompe volumétrique 3, qui le refoule dans le dialyseur 1, où il circule au contact d'une face de la membrane 2. Le sang épuré traverse ensuite un pièce à bulles 4 et il est réinjecté dans une veine V du patient.

Un liquide de dialyse ou dialysat circule dans le dialyseur 1 au contact de l'autre face de la membrane 2.

Un liquide chargé d'une partie des impuretés qui se trouvent dans le sang et qui auraient dû être épurées par les reins, notamment d'urée et de créatinine, passe à travers la membrane et se mélange au liquide de dialyse.

L'appareil selon la figure 1, est conçu pour être un appareil portatif léger, qu'un patient peut utiliser chez lui et amener en déplacements.

Il comporte un réservoir ou conteneur 5, qui contient le liquide de dialyse et qui a une capacité de l'ordre de 10 litres. Le réservoir 5 est, de préférence, un réservoir en matière plastique, par exemple en polyéthylène, qui est composé de deux parties 5a, 5b emboîtées ou soudées hermétiquement l'une à l'autre. Une cartouche 7 de régénération du dialysat de tout type connu, est disposée verticalement au centre du réservoir 5, sur un socle 8, qui fait partie du réservoir et qui comporte un conduit axial 9 qui débouche au centre de la base de la cartouche 7.

La température du liquide de dialyse est maintenue entre 37°C et 38°C par des ceintures chauffantes thermostatées placées autour du conteneur sous une housse isolante.

Le dialysat 6 est prélevé dans le fond du réservoir 5 par une canalisation d'aspiration 10 qui est munie d'un clapet anti-retour 11. Le dialysat traverse le dialyseur dans lequel il circule à contre-courant par rapport au sang. Il ressort du dialyseur par une canalisation 12, qui est un tube souple qui passe à travers la pompe volumétrique 3. La pompe 3 refoule le dialysat dans un tube souple 13, qui pénètre dans le réservoir 5 et qui est connecté sur le canal 9. On voit donc que le dialysat est recyclé en permanence. Le dialysat chargé d'impuretés qui sort du dialyseur 1 pénètre à la base de la cartouche de régénération 7 et traverse celle-ci de bas en haut en s'épurant. Il sort épuré par l'extrémité supérieure de la cartouche et il retombe en pluie dans le fond du réservoir 5.

Avantageusement, le réservoir 5 et la cartouche filtrante 7 forment une unité jetable après une séance d'hémodialyse.

L'appareil d'hémodialyse comporte certains dispositifs connus de mesure et d'alarme. Par exemple, le piège à bulles 4 est équipé de dispositifs pour éviter les risques d'hémorragie ou d'embolie gazeuse.

Il comporte, de façon connue, un couple émetteur-récepteur d'ultrasons placés de part et d'autre du bas du piège à bulles 4. Si le piège à bulles se remplit d'air qui risquerait d'être entraîné dans la veine V et de provoquer une embolie gazeuse, les ultrasons ne sont plus transmis et le détecteur à ultrasons déclenche une alarme et un arrêt automatique de la pompe 3.

Le piège à bulles 4 est également équipé d'un détecteur colorimétrique d'hémoglobine qui comporte un récepteur photo-électrique monochromatique réglé sur la couleur rouge de l'hémoglobine du sang.

Ce détecteur déclenche une alarme et un arrêt automatique si la couleur rouge s'éclaircit ou disparaît, ce qui correspond à une baisse de niveau du sang dans le piège à bulles.

En fin de dialyse, on branche un sachet de sérum physiologique sur le circuit de sang pour le rincer. Lorsque le sérum arrive dans le piège à bulles, le détecteur colorimétrique arrête automatiquement la pompe 3.

Le circuit de dialysat comporte également un détecteur colorimétrique d'hémoglobine qui détecte la présence de sang dans le dialysat en cas de déchirure de la membrane 2 et qui déclenche une alarme et un arrêt automatique de la pompe 3.

Tous ces appareils de contrôle circulatoire sont connus et n'ont pas été représentés sur le dessin.

Les appareils d'hémodialyse connus comportent deux pompes volumétriques distinctes, l'une pour faire circuler le sang et l'autre pour faire circuler le liquide de dialyse. Cette solution conduit à des appareils lourds et encombrants.

Un appareil selon l'invention comporte une seule pompe volumétrique 3, de type péristaltique, c'est-à-dire une pompe comportant un rotor qui est excentré par rapport à un berceau circulaire et sur lequel sont montés des galets 14 qui écrasent des tubes souples, qui passent entre les galets et le berceau. Le sang et le liquide de dialyse circulent dans deux tubes souples distincts de diamètre interne différent, qui sont écrasés par un rotor de pompe unique, de telle sorte que le rapport entre les débits de sang et de liquide de dialyse reste constant, quelle que soit la vitesse de la pompe 3.

Le compartiment du dialyseur dans lequel circule le sang est connecté sur un conduit de refoulement 15 de la pompe 3, tandis que le compartiment du dialyseur dans lequel circule le dialysat est connecté sur un conduit 12 d'aspiration de la pompe 3. Ainsi, il existe une différence positive entre la pression relative du sang et la pression relative du dialysat, de part et d'autre de la membrane 2.

Un des paramètres essentiel d'une dialyse est le volume d'ultrafiltrat (eau + sels) qui passe à travers la membrane par unité de temps et qui détermine la perte de poids d'un patient pendant une durée de dialyse déterminée.

Selon une caractéristique essentielle, un appareil de dialyse selon l'invention, comporte des moyens pour faire varier automatiquement la pression du liquide de dialyse dans le dialyseur afin de faire varier la différence de pression entre le sang et le liquide de dialyse, de part et d'autre de la membrane 2, pour obtenir une perte de poids par unité de temps déterminée.

Une canalisation 16 est connectée en dérivation sur la canalisation d'aspiration 10, entre le clapet anti-retour 11 et le dialyseur 1. Cette canalisation, qui est un tube souple, passe dans une pompe péristaltique 17 et elle retourne au réservoir 5 dans le bas de celui-ci.

En variante, la canalisation 16 sortant de la pompe 17 peut être rebranchée sur la conduite 10 en amont du clapet 11. La pompe 17 peut être entraînée dans un sens ou dans l'autre.

L'appareil comporte un premier capteur de pression 18 qui mesure la pression relative du sang au niveau du dialyseur ou à la sortie de celui-ci, par exemple dans le piège à bulles 4. Il comporte un deuxième capteur de pression 19 qui mesure la pression relative du dialysat au niveau du dialyseur ou à la sortie de celui-ci. Les signaux délivrés par les capteurs 18 et 19 sont transmis à un dispositif de régulation 20 qui comporte une boucle de régulation qui commande automatiquement le sens et la vitesse de rotation de la pompe 17.

Une membrane de dialyse déterminée a une perméabilité donnée par le constructeur qui correspond au volume de liquide qui traverse la membrane par unité de différence de pression transmembranaire et par unité de temps. Cette perméabilité varie en général entre 0,03 et 0,06 ml/Pa/heure.

Pour obtenir une perte de poids par unité de temps déterminée un premier moyen selon l'invention consiste à calculer, à partir de la perméabilité de la membrane, la pression transmembranaire nécessaire pour obtenir cette perte de poids.

Le régulateur 20 comporte des moyens permettant d'introduire la valeur calculée comme pression de consigne dans la boucle de régulation. A partir de là, le régulateur 20 calcule à tout instant la différence algébrique entre les pressions relatives mesurées par les capteurs 18 et 19 et la compare à cette valeur de consigne et il fait varier automatiquement la pression du dialysat dans le dialyseur en faisant varier le sens et la vitesse de rotation de la pompe 17 pour maintenir la différence de pression égale à la valeur de consigne.

Si la différence de pression mesurée devient inférieure à la valeur de consigne, il faut réduire la pression relative du dialysat. Le régulateur 20 commande alors la mise en route de la pompe 17, dans le sens des aiguilles d'une montre, de sorte que la pompe 17 prélève une partie du dialysat ayant franchi le clapet anti-retour et le renvoie vers le réservoir 5, ce qui réduit la pression du dialysat dans le dialyseur 1 et accroît donc la différence de pression transmembranaire. Si la différence de pression reste inférieure à la valeur de consigne, le régulateur 20 accroît la vitesse de rotation de la pompe 17 jusqu'à ce que l'écart entre la différence de pression mesurée par les capteurs 18 et 19 et la valeur de consigne soit annulé et maintient ensuite la vitesse de la pompe 17 constante.

Si au contraire, la différence de pression mesurée est supérieure à la valeur de consigne, il faut augmenter la pression relative du dialysat. Dans ce cas, le régulateur 20 commande la mise en route de la pompe 17 dans le sens contraire aux aiguilles d'une montre et fait croître la vitesse jusqu'à ce que l'écart soit annulé.

La figure 1 représente un appareil qui comporte un capteur de poids 21 qui mesure la variation de poids du réservoir 5 par unité de temps. Cette variation de poids correspond à la variation du poids de liquide et de sels contenus dans le réservoir 5, c'est-à-dire à la perte de poids par unité de temps du patient.

Le signal émis par le capteur 21 est transmis au régulateur 20 qui comporte une deuxième boucle de régulation qui compare la variation de poids mesurée à une valeur de consigne et qui agit automatiquement sur le sens et sur la vitesse de rotation de la pompe 17 pour faire varier la pression du dialysat dans le dialyseur, afin de ramener à zéro l'écart entre la variation de poids mesurée et la valeur de consigne.

La figure 1 représente un mode de réalisation d'un capteur de poids dans lequel le conteneur 5 est monté sur le plateau d'une balance électronique qui émet un signal électrique proportionnel au poids total du conteneur. Dans ce cas, le régulateur 20 comporte un circuit différentiateur, qui émet un signal proportionnel à la variation de poids par unité de temps ou une unité de calcul qui calcule cette variation.

Le régulateur 20 peut être constitué par des boucles de régulation analogiques ou par un microprocesseur qui reçoit les signaux émis par les capteurs 18, 19 et 21, qui les traite et qui émet un signal de commande de la pompe 17.

La figure 1 représente un mode de réalisation préférentiel d'un appareil qui comporte une double régulation de la pression du dialysat dans le dialyseur, soit à partir de la différence despressions mesurées par les capteurs 18 et 19, soit à partir de la mesure de la variation de poids du conteneur mesurée par le capteur 21. Dans ce cas, l'appareil est équipé d'un commutateur qui permet de choisir l'une ou l'autre des deux régulations.

En variante, un appareil selon l'invention peut comporter une seule des deux régulations.

La figure 1 représente un mode de réalisation comportant deux capteurs de pression 18 et 19. En variante, les deux capteurs de pression 18 et 19 peuvent être remplacés par un capteur de pression différentielle qui mesure directement la différence de pression transmembranaire.

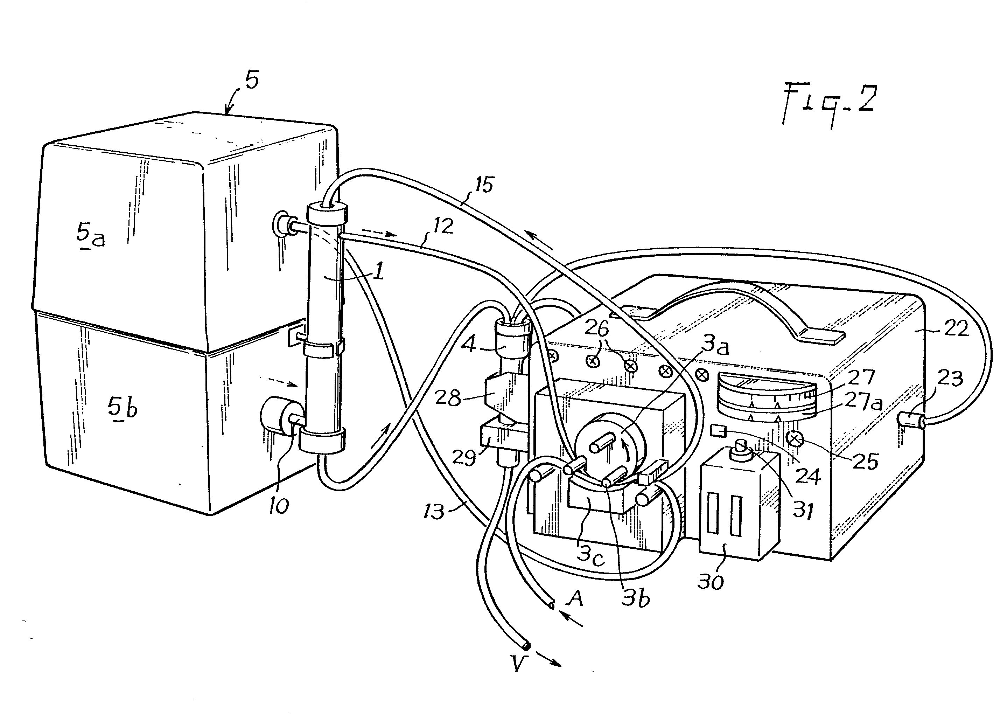

La figure 2 représente une vue d'ensemble en perspective d'un appareil d'hémodialyse portatif selon l'invention.

Cet appareil comporte, d'une part, un réservoir 5 contenant une cartouche de régénération qui constitue une unité jetable. Il comporte un dialyseur 1 qui est également jetable après usage. Il comporte un coffret portatif 22 qui contient les appareils d'assistance circulatoire et de mesure. Ces composants sont reliés entre eux par des tubes souples dans lesquels circulent le sang et le liquide de dialyse.

0187109

La figure 2 représente une vue en perspective d'un mode de réalisation d'un coffret portatif 22.

Ce coffret contient la pompe volumétrique 3, dont on voit le rotor 3a portant trois galets 3b. On voit également les deux tubes souples 13 et 15 dans lesquels circulent respectivement le liquide de dialyse et le sang, qui sont écrasés entre les galets 3b et le berceau circulaire 3c de la pompe.

Le coffret 22 comporte des embouts 23 dont un seul est visible sur le dessin, qui est celui sur lequel on connecte un tube souple de prise de pression sanguine.

Le coffret 22 porte, sur sa face avant, un bouton-poussoir 24 de marche-arrêt et une lampe 25 de signalisation de fonctionnement. Il porte également cinq lampes d'alarme 26 qui indiquent respectivement une fuite de sang dans le dialysat, une détection d'air dans le piège à bulles, une détection de changement de couleur du liquide dans le piège à bulles, une variation anormale de la pression sanguine et une variation anormale de la pression transmembranaire.

Le repère 27 représente un galvanomètre qui affiche la mesure de la pression du sang et qui comporte deux index, l'un de pression maxima et l'autre de pression minima.

Le repère 27a représente un deuxième galvanomètre qui affiche la pression transmembranaire.

Le coffret 22 porte sur une de ses faces latérales une sonde à ultrasons 28 et un détecteur colorimétrique d'hémoglobine 29 en forme de pinces, disposés l'un au-dessus de l'autre. Un piège à bulles 4 transparent, est engagé dans la pince formée par la sonde 28 et le détecteur colorimétrique 29 qui font fonction de support du piège à bulles.

Le repère 30 représente un interrupteur manuel-automatique qui sert à mettre en service ou hors service la régulation automatique de perte de poids.

Le repère 31 représente un bouton-poussoir qui sert à supprimer l'alarme tant qu'il est maintenu par le malade.

Le coffret 22 contient, en outre, la deuxième pompe péristaltique 17 non visible sur le dessin ainsi que les circuits et composants électroniques du régulateur 20 et les capteurs de pression.

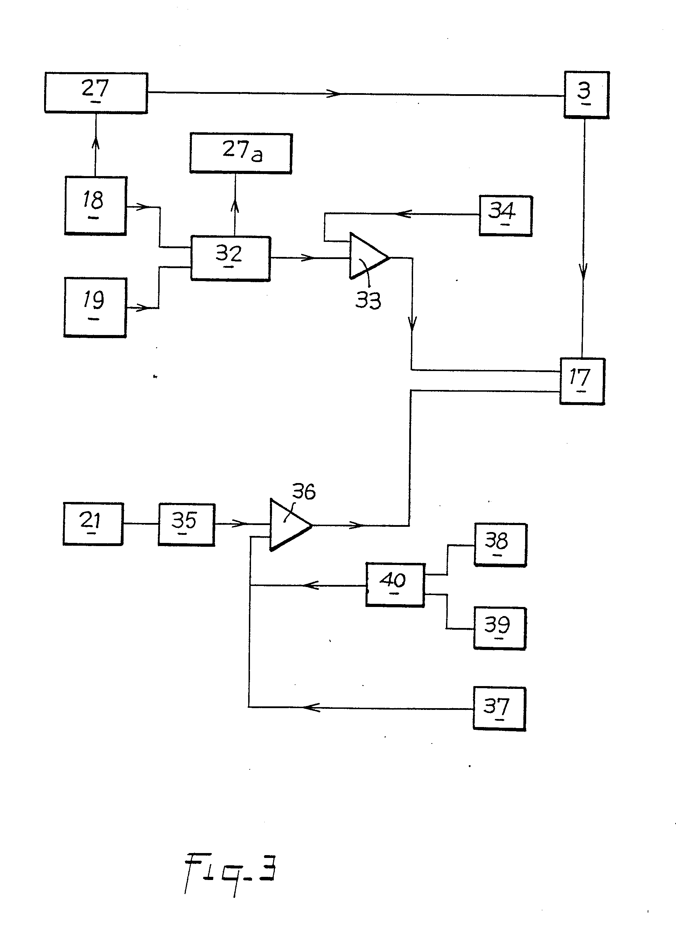

La figure 3 est un schéma d'un mode de réalisation des circuits électroniques de régulation de la perte de poids par unité de temps.

Les repères 3 et 17 représentent les contacteurs des moteurs des deux pompes péristaltiques.

Les repères 18 et 19 représentent respectivement le capteur de pression sanguine et le capteur de la pression du dialysat. Le repère 27 est le galvanomètre à contacts représenté sur la figure 2 qui affiche la pression du sang. Si celle-ci atteint l'un des seuils fixés par les deux contacts, la pompe 3 est arrêtée automatiquement.

Le repère 32 représente un circuit qui fait la différence algébrique entre les signaux délivrés par les capteurs 18 et 19, cette différence correspondant à la pression transmembranaire.

Le repère 27a représente un galvanomètre ou un voltmètre numérique, qui affiche la pression transmembranaire. Le repère 33 représente un comparateur qui compare la pression transmembranaire mesurée à une valeur de consigne qui est entrée manuellement par le patient dans un registre d'entrée 34 qui peut être par exemple un potentiomètre, dans le cas d'une installation analogique ou bien un clavier dans le cas d'une installation comportant un microprocesseur. Le comparateur 33 délivre un signal dont la polarité correspond au sens de l'écart entre la pression transmembranaire calculée et la valeur de consigne et dont l'intensité correspond à la valeur de cet écart. Ce signal commande automatiquement le sens de marche et la vitesse de la pompe volumétrique 17.

La partie supérieure du schéma qui vient d'être décrite concerne la régulation de la perte de poids au moyen d'une pression transmembranaire maintenue constamment égale à une valeur de consigne calculée et introduite dans l'appareil avant le début d'une dialyse.

La partie inférieure du schéma concerne la régulation de la perte de poids par unité de temps par mesure directe de cette perte de poids.

Le repère 21 représente un capteur de poids qui mesure le poids global du réservoir 5. Le repère 35 représente un circuit différentiateur qui émet un signal proportionnel à la variation de poids par unité de temps. Le repère 36 représente un comparateur qui compare la variation de poids mesurée à une valeur de consigne.

La variation de poids par unité de temps désirée peut être calculée par le patient et introduite manuellement dans un registre d'entrée 37.

En variante, dans le cas où l'appareil comporte une unité de calcul, on peut introduire dans un registre 38 la perte de poids total à atteindre au cours d'une séance de dialyse et dans un registre 39 la durée de la dialyse. Dans ce cas, le calculateur 40 calcule la perte de poids par unité de temps désirée et délivre la valeur calculée comme valeur de consigne au comparateur 36.

Le comparateur 36 émet un signal dont la polarité et l'intensité sont proportionnels au sens et à la valeur de consigne et ce signal commande le fonctionnement de la pompe 17, dans le sens et à la vitesse qui tend à annuler cet écart.

Les circuits de régulation de la perte de poids du patient sont des circuits qui comportent des boucles de régulation qui peuvent être réalisées en circuits analogiques ou par des moyens numériques dans le cas où l'appareil comporte un microprocesseur. La pompe péristaltique 3 est asservie à la pression veineuse. Si celle-ci atteint l'un des deux seuils fixés par les index du galvanomètre 27, la pompe 3 s'arrête automatiquement ainsi que la pompe 17.

REVENDICATIONS

1. Dispositif d'hémodialyse du type comportant un dialyseur (1) équipé d'une membrane de dialyse (2), un réservoir (5) contenant le liquide de dialyse (6), une cartouche (7) de régénération du liquide de dialyse et des moyens pour faire circuler le sang d'un patient à travers ledit dialyseur et pour faire circuler ledit liquide de dialyse en circuit fermé entre ledit réservoir (5), ledit dialyseur (1) et ladite cartouche de régénération (7), caractérisé en ce que le conduit (10) de liquide de dialyse allant dudit réservoir (5) au dialyseur (1) comporte un clapet anti-retour (11) et une dérivation (16) qui est branchée sur ledit conduit, en aval dudit clapet et qui comporte une première pompe volumétrique (17) à double sens de marche.

2. Dispositif d'hémodialyse selon la revendication 1, caractérisé en ce que lesdits moyens pour faire circuler le sang et le liquide de dialyse sont constitués par une même deuxième pompe volumétrique (3), de type péristaltique dont le rotor écrase deux tubes souples (13, 15) qui véhiculent respectivement le sang et ledit liquide de dialyse, avec des débits constamment proportionnels entre eux et le tube (15) amenant le sang à l'entrée du dialyseur (1) est connecté sur le refoulement de ladite pompe (3), tandis que le tube (12), qui reprend le liquide de dialyse à la sortie du dialyseur, est connecté sur l'aspiration de ladite pompe (3), de sorte qu'il existe une différence positive entre la pression du sang et la pression du liquide de dialyse, de part et d'autre de la membrane de dialyse.

3. Dispositif selon l'une quelconque des revendications 1 et 2, comportant un premier capteur de pression (18) qui mesure la pression du sang dans le dialyseur (1) ou à la sortie de celui-ci et un deuxième capteur de pression (19) qui mesure la pression du liquide de dialyse dans le dialyseur (1) ou à la sortie de celui-ci, caractérisé en ce qu'il comporte une boucle de régulation qui comporte des moyens (32) pour faire la différence entre les signaux délivrés par les deux capteurs (18, 19) et un comparateur (33) qui compare cette différence à une valeur de consigne (34) et qui émet un signal proportionnel à l'écart entre la différence mesurée et la valeur de consigne, qui agit automatiquement sur le sens et sur la vitesse de rotation de ladite première pompe volumétrique (17) afin de corriger la pression du liquide de dialyse dans le sens qui maintient ladite

2    0187109

différence constamment égale à ladite valeur de consigne.

4. Dispositif selon l'une quelconque des revendications 1 et 2, caractérisé en ce que ladite cartouche de régénération (7) du liquide de dialyse est placée à l'intérieur dudit réservoir (5), qui est connecté au reste de l'appareil par des tubes souples et ledit dispositif comporte un capteur de poids (21, 35) qui mesure la variation de poids dudit réservoir par unité de temps et une boucle de régulation comportant un comparateur (36) qui compare ladite mesure à une valeur de consigne (37) et qui émet un signal proportionnel à l'écart, lequel signal agit automatiquement sur le sens et sur la vitesse de rotation de ladite première pompe (17) afin de corriger la pression du liquide de dialyse dans le sens qui maintient la perte de poids mesurée par unité de temps constamment égale à ladite valeur de consigne.

0187109

Fig-1

Fig. 2

5
5a
5b
10
1
4
28
29
13
3c
3b
A
V
15
12
26
3a
27
27a
24
25
31
30
22
23

2/3

0187109

$F_{iq-3}$

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-2 745 347 (GAMBRO A.B.) <br> * Figure 1 * | 1 | A 61 M 1/16 |
| A | FR-A-2 520 621 (INTERMEDICAT GmbH) <br> * Figure * | 3 | |
| A | FR-A-2 397 197 (S.O.D.I.P.) <br> * Figure 2; page 7, ligne 35 - page 8, ligne 25 * | 4 | |
| A | FR-A-2 261 018 (RENAL SYSTEMS INC.) | | |

DOMAINES TECHNIQUES RECHERCHES (Int. Cl.4)

A 61 M

Le present rapport de recherche a été établi pour toutes les revendications

| Lieu de la recherche | Date d achèvement de la recherche | Examinateur |
|---|---|---|
| LA HAYE | 11-03-1986 | VEREECKE A. |